# EUROPEAN PATENT SPECIFICATION

(11) **EP 1 670 443 B1**
(45) Date of publication and mention of the grant of the patent: **07.05.2014**
(21) Application number: 04769700.8
(22) Date of filing: 08.10.2004
(51) Int. Cl.: A61K 9/72, A61K 31/67

(54) **AEROSOL FORMULATION COMPRISING FORMOTEROL IN SUSPENSION**
AEROSOL-FORMULIERUNG MIT FORMOTEROL IN SUSPENSION
AMELIORATIONS APPORTEES A DES COMPOSES ORGANIQUES

(30) Priority: 09.10.2003 GB 0323685
(43) Date of publication of application: 21.06.2006
(73) Proprietor: Jagotec AG, 4132 Muttenz (CH)
(72) Inventor: MUELLER-WALZ, Rudi, 79650 Schopfheim (DE)
(74) Representative: Mintz Levin Cohn Ferris Glovsky and Popeo LLP
(86) International application number: PCT/IB2004/003465
(87) International publication number: WO 2005/034927

(56) References cited:
- WO-A-00/48587
- WO-A-03/074024
- WO-A1-03/086349
- US-A- 5 709 884
- US-A- 6 054 488
- US-A1- 2002 018 753
- US-B1- 6 475 467
- PHARMAEUROPA, vol. 14, no. 4, 4 October 2002 (2002-10-04), pages 693-696,

## Description

The present invention relates to pharmaceutical aerosol formulations comprising formoterol fumarate di-hydrate in suspension, which formulations are capable of being dispensed from a metered dose inhaler device with good Delivered dose uniformity and with a high fine particle fraction. In particular, the invention relates to such formulations additionally comprising a steroid in solution. The invention also relates to a method of producing such formulations.

Metered dose inhaler (MDI) formulations are well known in the art. They typically consist of suspensions or solutions of an active substance in a propellant or mixture of propellants, and contain other optional ingredients such as solvents and surfactants and preservatives. MDI formulations are stored in suitable pressurized containers that are equipped with a valve to permit an active substance to be dispensed on demand. In common with all drug products, they are subject to regulatory review as to their safety and efficacy before they can be marketed for use in humans. However, unlike oral or injectable products, which typically contain a single dosage form, an aerosol formulation for use in an MDI may contain multiple doses, e.g. tens or even hundreds of doses in a single container, and each of these must be delivered with a uniform delivered dose, and reliable particle size uniformity. Furthermore, MDI formulations must be capable of delivering doses uniformly even after long storage periods, e.g. 2 to 3 years, under harsh conditions of temperature and humidity in order to mimic all manner of patient-use conditions.

Formoterol fumarate di-hydrate has proven to be a particularly recalcitrant material to formulate. When formulated as suspended particles in aerosol formulations, the particles are prone to agglomeration, and to form sediments which are not readily re-dispersible. Furthermore, the particles often adhere to the inner surface of both canisters and valves. As a result, such formulations often display irregular dosing.

US 6,054,488 addresses formulating formoterol fumarate as the sole active substance in suspension aerosol formulations in MDIs. This reference describes difficulties in formulating formoterol, in particular the problem of deposition of the suspended particles on canisters and valves leading to poor dose reproducibility. Often surface active agents, or other adjuvants need to be added to such formulations to counteract these problems. However, because most acceptable propellants are poor solvents for these surfactants and other adjuvants, one needs to use polar co-solvents to assist in their dissolution. Various polar co-solvents have been employed, but ethanol is a particularly useful co-solvent in this regard. Notwithstanding the benefits that ethanol can confer on suspension formulations, care must be exercised to ensure one does not add too much ethanol in order to avoid certain undesired effects associated with its use. In fact, US6,054,488 informs the skilled person that formulations containing an HFA and ethanol are extremely sensitive to the amount of ethanol employed. Nevertheless, '488 goes on to teach that if ethanol is employed in small amounts, i.e. up to 5% and preferably at lower amounts, i.e. 1.5% to 3.5% by weight, formoterol suspension formulations can be formed that exhibit substantially no growth in particle size or change in crystal morphology of the drug. Furthermore, such formulations are re-dispersible, and upon re-dispersion do not flocculate so quickly as to prevent reproducible dosing of the drug.

US 6,120,752 discloses medicinal aerosol formulations of the steroid, ciclesonide. Ciclesonide, it is stated, is advantageously employed as a solution in HFA and ethanol. The ethanol is provided to ensure that the active agent is fully dissolved and so it is added in relatively high amounts. Accordingly, whereas, this reference discloses that ethanol may be present in amounts of 3 to 25%, the preferred level is 8% and all of the examples use 5% or more, indeed, most employ 20% ethanol.

Both of these prior art references employ ethanol as a stabilizing excipient. However, they use it for different reasons, and the preferred levels taught in one reference would appear to be incompatible for formulating the active substance in the other reference. The art teaches, that stabilising amounts of ethanol useful in formoterol suspension formulations may not be appropriate or optimal for other active substances that might otherwise be usefully formulated in combination with formoterol.

The applicant has now found an alternative method of stabilizing formoterol fumarate di-hydrate aerosol suspensions. Furthermore, the method can be employed to form stable formulations even though they may contain, additionally, a steroid in solution.

Accordingly, the invention provides in a first aspect a pharmaceutical aerosol formulation for use in a metered dose inhaler (MDI) comprising formoterol fumarate di-hydrate in suspension, a propellant and ethanol, wherein the formoterol fumarate di-hydrate is provided as suspended particles having a water content of about 4.8 to 4.28%, more particularly 4.50 to 4.28% by weight.

The invention provides in a second aspect a pharmaceutical aerosol formulation for use in a metered dose inhaler (MDI) comprising formoterol fumarate di-hydrate in suspension, and a steroid in solution, a propellant and ethanol, wherein the formoterol fumarate di-hydrate is provided as suspended particles having a water content of about 4.8 to 4.28%, more particularly 4.50 to 4.28% by weight.

Formulations according to the present invention can be filled into canisters to form highly stable suspensions for use in MDI devices. Formulations exhibit substantially no particle growth or change of morphology of the suspended particles. There is also no, or substantially no, problem of deposition of the suspended particles on the surface of either canisters or valves, and so the formulations can be discharged from a suitable MDI device with high Delivered dose uniformity.

Formulations of the present invention meet Compendial requirements as to Delivered dose uniformity as set forth, for example in the United States and European Pharmacopoeias. For example, formulations of the present invention meet the requirement set out in the USP26-NF21 chapter <601> "Delivered dose Uniformity". Indeed, the formulations appear to be so stable that they may even meet the relatively more stringent Delivered dose uniformity requirements set forth in the current Draft Guidance from the FDA, published by the CDER in October 1998.

Accordingly, the invention provides in a third aspect a pharmaceutical aerosol suspension formulation for use in a metered dose inhaler (MDI) comprising formoterol fumarate di-hydrate in suspension, and optionally a steroid in solution, a propellant and ethanol, wherein the formoterol fumarate di-hydrate is provided as suspended particles having a water content of about 4.8 to 4.28%, more particularly 4.50 to 4.28% by weight, and wherein the formulation is capable of being dispensed from an MDI to provide a Delivered dose of formoterol fumarate di-hydrate that has a variance of no more than +/- 25%, of the mean Delivered dose when the formulation is stored at, 25 degrees centigrade and 60 relative humidity (rh), more particularly 40 degrees centigrade and 75% rh for up to 6 months, e.g. 1, 3 and 6 months.

Still further, the Delivered dose of the formulations contains a high fraction of fine particles, i.e. particles that are capable of penetrating the deep lung, e.g. having a diameter of less than about 5.8, more preferably less than about 4.7 microns.

Accordingly, in a fourth aspect, the invention provides a pharmaceutical aerosol suspension formulation for use in a metered dose inhaler (MDI) comprising formoterol fumarate di-hydrate in suspension, and optionally a steroid in solution, a propellant and ethanol, wherein the formoterol fumarate di-hydrate has a water content of about 4.8 to 4.28%, more particularly 4.50 to 4.28% by weight, and wherein the formulation is capable of being dispensed from a MDI to provide an Delivered dose of formoterol fumarate di-hydrate with a fine particle fraction of about 30 to 70%.

When a steroid is present in a formulation according to the invention, it is provided in solution and not in suspended form. As such, it exhibits none of the problems of flocculation or deposition that can lead to poor uniformity in terms of the Delivered dose. In fact, given that the formulations of the present invention employ sufficient ethanol to dissolve the steroid, any variance in the Delivered dose of the steroid occurs not as a result of any properties inherent to the formulation, but due only to any inaccuracies in the dosing attributable to the valve. However, applicant has found that given the high stability of the suspension, the formulation can be discharged using any commercially available valve means with highly precise shot weight (the total amount of material discharged from a MDI device). Given that the valves act with precision, applicant has found that the Delivered dose of steroid also can meet with Compendial requirements, and the Draft FDA Guidance referred to above.

Thus, the invention provides in a fifth aspect a pharmaceutical aerosol suspension formulation for use in a metered dose inhaler (MDI) comprising formoterol fumarate di-hydrate in suspension, and a steroid in solution, a propellant and ethanol, wherein the formoterol fumarate di-hydrate is provided as particles having a water content of about 4.8 to 4.28%, more particularly 4.50 to 4.28% by weight, suspended in the propellant and solvent, and wherein the formulation is capable of being dispensed from an MDI to provide an Delivered dose of the steroid that has a variance of no more than +/- 25%, of the mean Delivered dose when the formulation is stored at, 25 degrees centigrade and 60 % rh, more particularly 40 degrees centigrade and 75% rh for up to 6 months, e.g. 1, 3 and 6 months.

In a sixth aspect of the invention there is provided a pharmaceutical aerosol suspension formulation for use in a metered dose inhaler (MDI) comprising formoterol fumarate di-hydrate in suspension, and a steroid in solution, a propellant and ethanol, wherein the formoterol fumarate di-hydrate has a water content of about 4.5 to 4.28% by weight, wherein the formulation is capable of being dispensed from an MDI to provide an Delivered dose of steroid containing a fine particle fraction of about 30 to 70%.

Formulations of the present invention may be made by a process, which forms a seventh aspect of the invention, and comprises the step of drying the formoterol fumarate di-hydrate to a water content of about 4.8 to 4.28%, more particularly 4.50 to 4.28% by weight, before mixing the active ingredients with propellant and ethanol in a container according to techniques generally known in the art.

Formoterol fumarate di-hydrate raw material typically contains a certain amount of water in addition to the water of crystallization. Typically, the raw material is used directly in formulations. However, applicant found that by subjecting the raw material to a drying step that is designed to drive off all, or substantially all, of the residual water but not the water of crystallisation, formulations of very high stability can be achieved. Applicant found that drying to a water content of about 4.8 to 4.28%, more particularly 4.5 to 4.28% enabled the preparation of suspension formulations with good stability. The drying step is carried out under conditions of pressure and temperature to achieve the desired water content within a time that is both practical and economical. The skilled person will appreciate that the inventive concept resides in the realization that the material should be subjected to a drying step, to achieve the above stated preferred level of dryness, and not in the means or conditions by which the drying is achieved. Accordingly, consistent with the economic consideration, and the need to dry in a reasonably practical period of time, and consistent with the requirement of preserving the integrity of the active substance's water of crystallization, virtually any conditions of temperature and pressure can be employed.

Preferably however, the material can be dried at a temperature of between 10 and 70°C. Preferably, also, the material can be dried at a pressure of 10 to 400 mbar.

Water content is measured according to the Karl Fischer Method. The Karl Fischer method is a well known analytical tool for the measurement, specifically, of a sample's water content. It is a titrimetric method that involves the reaction between water contained in a sample and a Karl Fischer Reagent, which is a mixture of sulphur dioxide, iodine, pyridine and methanol. The preferred reagent is Hydranal Composite 1 or 5, wherein 1 is preferred. The reagent reacts with suspended and dissolved water. Furthermore, because the sample is dissolved during this method, it is also measures water of crystallization of a sample.

Whilst every precaution is taken to keep formulations dry, due to residual moisture from excipients and moisture ingress that might occur during conditions of storage and use, formulations of the present invention may contain small amounts of moisture. Preferably formulations of the present invention contain levels of moisture of 50 ppm to 800 ppm, more particularly 100 to 600 pm.

The Delivered dose of a formulation is the amount of active agent to achieve a therapeutic effect or prophylactic effect that is emitted from a MDI device upon actuation. Depending on the drug substance to be emitted, and the nature of the valve, the Delivered dose may be the amount of active material emitted upon a single actuation of the MDI, or it may be the amount emitted from two or more actuations. It is not a measure of the total amount of material (actives and excipients) that is emitted upon actuation. This is often referred to as the Shot Weight.

The Delivered dose may not only vary between different formulations of a batch; it may also vary within a given formulation when that formulation contains a plurality, e.g. 10 or even 100 or more doses of the active substance. Accordingly, the variance of the Delivered dose is typically measured for a given formulation by taking measurements at the beginning, middle and end of that formulation's life. In this way, a measure of the in-use variability in the dosing is obtained. Further, batches of formulations may be tested to obtain a picture of the inter-batch variability of a formulation after determined periods of storage. The variance of formulations according to the present invention is discussed further in the Examples. Variance, in both cases, must fall within limits proscribed by regulatory authorities if a product is to gain market authorisation. As stated herein above, formulations of the present invention fall within all the Compendial requirements for variance of Delivered dose, and can even meet the more stringent requirements referred to in the FDA Draft Guidance for Industry published in October 1998.

The fraction of active agent contained in the total Delivered dose that is of small enough aerodynamic diameter to reach the deep lung upon inhalation is often referred to as the fine particle fraction (or FPF) of the Delivered dose, and the absolute amount of fine particles emitted is often referred to as the Fine Particle Dose (or FPD). As stated herein above, formulations of the present invention are capable of being delivered with good Delivered dose uniformity and with a high FPF, both in relation to the formoterol fumarate di-hydrate, and the steroid.

The Delivered dose and its variance can be measured using the Dosage Unit Sampling Apparatus (DUSA). The FPF can be measured using an Andersen Cascade Impactor (ACI). The measurement methodology and the apparatus therefor are well known in the art, and are described in the United States Pharmacopoeia Chapter <601>, or in the inhalants monograph of the European Pharmacopoeia, both of which documents are hereby incorporated by reference. The USP states that the Apparatus 1 should be used for the measurement of FPF. The USP also states that Delivered dose Uniformity should be measured with DUSA or its equivalent. However, the Delivered dose and Delivered dose uniformity are preferably measured using the so-called Funnel Method. The Funnel Method is described in Drug Delivery to the Lungs, VIII p116 to 119*,* which is hereby incorporated by reference. In summary, the Funnel Method consists of discharging a formulation from a MDI into a Funnel Apparatus, which basically consists of a standard Buchner Funnel. The discharged dose is captured on the glass sinter of the Funnel, and can be washed off, and the dose determined using HPLC analysis. The Funnel Method gives comparable results to the standard USP apparatus, and is generally considered to be an equivalent of the DUSA apparatus.

Formoterol fumarate di-hydrate is a long acting, selective B-2-adrenoceptor agonist. It is well known in the literature and is the active substance in the commercially available product - Foradil (registered trademark). The skilled person will be fully apprised of its properties and uses, and no further discussion needs to be had here.

Formulations of the present invention may contain from 0.001 to 0.1%, more particularly 0.003 to 0.05%, still more particularly 0.005 to 0.02% by weight of formoterol fumarate di-hydrate.

Formoterol fumarate di-hydrate is a very potent material. The typical therapeutic or prophylactic dose of this material to be emitted from an MDI device will depend upon the patient, and the type and severity of the condition to be treated. The dose may vary from about 3 to 15 micro-grams, more particularly 6 to 12 micro-grams, e.g. 10 micrograms. In a finished form, a formulation will be packaged, and will be accompanied by labeling. The dose presented on the packaging and/or labeling of a finished form is often referred to as its Label Claim. In order to ensure inter-batch quality and reproducibility, the mean dose of formulation emitted from a MDI, should not vary considerably from the Label Claim. In this regard, given the good stability of the formulation of the present invention, the mean Delivered dose of formoterol fumarate di-hydrate does not fall outside a range of +/- 15% of the Label Claim.

Steroids for use in the present invention include any of the materials selected from the group consisting of budesonide, ciclesonide, mometasone, fluticasone, beclomethasone, flunisolide, loteprednol, triamcinolone, amiloride, rofleponide or a pharmaceutically acceptable salt or derivative of these active compounds, such as mometasone furoate, fluticasone dipropionate, beclomethasone dipropionate, triamcinolone acetonide or flunisolide acetate, where optically active, these materials can be used in the form of their active isomer or as an isomer mixture.

Particularly preferred steroids for use in the present invention are those that are reported in the art as having been advantageously formulated as solutions in a propellant, for example beclamethasone dipropionate, flunisolide, triamcinolone acetonide and ciclesonide.

An appropriate therapeutic or prophylactic Delivered dose for the steroids will depend upon the steroid selected, the patient and the type and severity of the condition to be treated. It may vary within a range of about 10 to 2000, more particularly 100 to 1600 micro-grams daily dose.

Taking ciclesonide as an example, this material may be present in a formulation to provide a Delivered dose of 50 to 400, more preferably 100 to 400 micro-grams per application.

Taking beclamethasone as an example, this material is usually administered in 50, 100, 200 and 250 micro-grams per puff. The recommended daily dose for children is 100 to 400 micro-grams, and 1000 to 1600 micro-grams for adults.

In respect of the steroid, the mean Delivered dose of formulations of the present invention does not fall outside a range of +/- 15% of the Label Claim of the steroid.

In a preferred embodiment of the present invention, a formulation as herein above defined additionally contains a cromone selected from the group consisting of a pharmaceutically acceptable salt of cromoglycinic acid, e.g. di-sodium cromoglycate and/or nedocromil. Both of these materials are pharmaceutically active substances, and so their use in the present invention is limited to sub-therapeutic or sub-prophylactic levels, e.g. from about 5 to 250 micrograms per puff of a MDI inhaler. The materials may be employed to afford the formulations protection against moisture. The use of these materials to protect moisture sensitive active substances is reported in US6,475,467.

Preferably, formulations of the present invention contain from 0.001 to 1%, more particularly 0.005 to 0.2%, still more particularly 0.01 to 0.1% by weight cromone, e.g. disodium cromoglycate.

Suitable propellants for use in the aerosol formulations according to the invention may be any of the pressure-liquefied propellants which customarily may find use in metered-dose aerosols, for example fluorochlorocarbons such as trichloromonofluoromethane (F11), dichlorodifluoromethane (F12), monochlorotrifluoromethane (F13), dichloro-monofluoromethane (F21), monochlorodifluoromethane (F22), monochloromonofluoromethane (F31), 1,1,2-trichloro-1,2,2-trifluoroethane (F113), 1,2-dichloro-1,1,2,2-tetrafluoroethane (F114), 1-chloro-1,1,2,2,2-pentafluoroethane (F115), 2,2-dichloro-1,1,1-trifluoroethane (F123), 1,2-dichloro-1,1,2-trifluoroethane (F123a), 2-chloro-1,1,1,2-tetrafluoroethane- (F124), 2-chloro-1,1,2,2-tetrafluoroethane (F124a), 1,2-dichloro-1,1-difluoroethane (F132b), 1-chloro-1,2,2-trifluoroethane (F133), 2-chloro-1,1,1-trifluoroethane (F133a), 1,1-dichloro-1-fluoroethane (F141b) and 1-chloro-1,1-difluoroethane (F142b), alkanes such as propane, butane and isobutane, fluorinated alkanes such as octafluoropropane (F218) and in particular hydrofluoroalkanes such as difluoromethane (HFA 32), pentafluoroethane (HFA 125), 1,1,2,2-tetrafluoroethane (HFA 134), 1,1,1,2-tetrafluoroethane (HFA 134a), 1,1,2-trifluoroethane (HFA 143), 1,1,1-trifluoroethane (HFA 143a), difluoroethane (HFA 152a), 1,1,1,2,3,3,3-heptafluoropropane (HFA 227) and the like.

Preferred propellants are the hydrofluoroalkanes of the general formula.

CxHyFz (I)

in which x is the number 1, 2 or 3, y and z are each an integer >==1 and y+z=2x+2.

Those hydrofluoroalkanes of the formula I in which x is the number 2 or 3 are particularly suitable.

Particularly preferred aerosol formulations are those which contain HFA 134 or HFA 227 or mixtures of these two propellants. HFA 134a and HFA 227 have a vapour pressure of about 6 bar and about 4.2 bar respectively at 20[deg.] C. Both propellants differ with respect to their density (about 1.2 g/ml for HFA 134a and about 1.4 g/ml for HFA 227), which is important insofar as it is possible by suitable choice of the propellant or propellant mixture to match its density better to the density of the suspended substances and thus to keep the latter better in suspension. If desired, the density of the propellant can be further reduced by addition of co-solvents or other propellants, such as ethanol, diethyl ether, propane, n-butane or isobutane.

The aerosol formulations according to the invention can preferably contain one or more hydrofluoroalkanes of the formula I, particularly preferably 1,1,1,2-tetrafluoroethane (HFA 134a) and/or 1,1,1,2,3,3,3-heptafluoropropane (HFA 227), and their proportion in the total formulation can preferably be at least about 50% by weight and particularly preferably at least about 80% by weight. As a rule, it is advantageous to employ these propellants in an amount of 90% by weight or more.

Ethanol is employed in the present invention in amounts sufficient to, along with the propellant, dissolve the steroid. Preferably, it is employed in amounts of no less than 5%, more particularly 5 to 8% by weight of the total formulation. Whereas the prior art teaches that this is a relatively high amount of ethanol to use in formulations containing formoterol in suspension, that could lead to stability problems, applicant found that by taking the precaution of carrying out the drying step described above, and optionally including a cromone, formulations of the present invention exhibit no, or substantially no, suspension stability problems.

The aerosol formulations according to the invention can contain no, or substantially no surfactant, i.e. contain less than approximately 0.0001% by weight of surface-active agents. This is particularly the case if one employs a cromone as described above. If desired, however, the formulations can contain surface-active agents conventionally employed in aerosol formulations, such as oleic acid, lecithin, sorbitan trioleate, cetylpyridinium chloride, benzalkonium chloride, polyoxyethylene (20) sorbitan monolaurate, polyoxyethylene (20) sorbitan monostearate, polyoxyethylene (20) sorbitan mono-oleate, polyoxypropylene/polyoxyethylene block copolymers, polyoxypropylene/polyoxyethylene/ethylenediamine block copolymers, ethoxylated castor oil and the like, where the proportion of surface-active agents, if present, can preferably be about 0.0001 to 1% by weight, in particular about 0.001 to 0.1% by weight, based on the total formulation.

Other optional adjuvants can be employed in formulations according to the present invention. For example, if desired, they can contain buffer substances or stabilizers such as citric acid, ascorbic acid, sodium EDTA, vitamin E, N-acetylcysteine and the like. In general, such substances, if present, are used in amounts of not more than approximately 1% by weight, for example in an amount of from approximately 0.0001 to 1% by weight, based on the total formulation.

Formulations according to the present invention are prepared by a process comprising a first step of drying formoterol fumarate di-hydrate raw material to water- content as described above. If a cromone is to be employed in the formulation, preferably it too is subjected to a similar drying step. After drying, these components can be weighed and mixed with a steroid in an aerosol vial.

A valve can then be crimped onto the vial, and a pre-mix of propellant and ethanol can be introduced through the valve under pressure. The whole mixture can then be placed in an ultra-sonic bath to form a solution of steroid, and a suspension of formoterol fumarate di-hydrate.

The vials may be filled with sufficient formulation to provide a plurality of dosages. Typically formulations may contain 50 to 150 dosages, more particularly 100 to 150 dosages. The formulations are typically filled with an overage of doses to avoid a situation where a patient could, under the proper conditions of use, actuate its MDI and find that there are no remaining doses to be delivered.

The vials or canisters used to contain the formulations according to the invention may be of plastics, metal or glass construction. It is a feature of the stability of the formoterol suspension of the inventive formulations that they exhibit no, or substantially no, tendency to deposit on the surface of the containers into which they are filled. This gives the formulator the latitude to choose from any of the commercially available alternatives, which can be advantageous from an economic view point. It is often the case with suspension aerosol formulations that special containers must be used in order to avoid stability problems, for example, those coated internally with special low surface energy coating materials, see for example US6,596,260.

Valves used with vials may by any of the standard metered dose valves available in the art. Typically, metered dose valves of 20 to 150 micro-litres can be employed. Often the Delivered dose of one or more active substance cannot be achieved with a single actuation of an MDI. It is preferred that, having regard to the active ingredients that are contained in the formulation, and having regard to the respective Label Claims, one chooses a valve that is capable of metering the dose within only one or two actuations, or puffs.

Formulations of the present invention find use as medicinal aerosol preparations for the treatment of disease states of the lung, for example asthma, e.g. mild, exercise-induced, moderate and severe bronchial-induced asthmas, cold air-induced asthma, COPD, and interstitial lung disease sarcoidosis.

In an embodiment of the present invention, formulations are provided containing a plurality of doses of formoterol fumarate di-hydrate, each dose containing 3 to 15 micro-grams, and a plurality of doses of beclamethasone, each dose containing 100 to 1600 micro-grams. Said formulations are suitable for the treatment of any of the aforementioned conditions.

In another embodiment of the present invention formulations are provided containing a plurality of doses of formoterol fumarate di-hydrate, each dose containing 3 to 15 micro-grams, and a plurality of doses of ciclesonide, each dose containing 50 to 400 micro-grams. Said formulations are suitable for the treatment of any of the aforementioned conditions.

Preferred features of the second and subsequent aspects of the invention are as for the first aspect *mutatis mutandis.*

There now follows as series of examples that serve to illustrate the invention.

### Example 1

The following formulation was prepared (FF denotes formoterol fumarate di-hydrate; BDP represents beclamethasone dipropionate. DSCG represents disodium cromoglycate).

| | % by weight |
|---|---|
| FF | 0.0086 |
| BDP | 0.0781 |
| Abs. Ethanol | 7.500 |
| HFA 227 | 92.369 |
| DSCG | 0.3430 |
| Oleic acid | 0.0100 |

Formoterol Fumarate Di-hydrate is dried at 20 to 40°C and at a maximum of 100mbar reduced air pressure.

DSCG is dried at 80°C and a maximum 100mbar to a water content of less than 4%.

The dried materials are deposited along with the beclamethasone dipropionate in a filling vessel and the vessel is evacuated to less than 100 mbar air pressure.

Absolute ethanol and pharmaceutical grade HFA 227 are pre-blended in another container. The blending container is then connected to the filling vessel and the blend is fed into the vessel. The resultant filled vessel is homogenised for 30 minutes at 300 rpm.

A 14mm plain aluminium container (Presspart Manufacturing, Blackburn, England), is crimped around a 50 micro-litre valve (Valois Pharm SA, France).

An aliquot from the filling vessel is pressure-filled into the aluminium can in a quantity sufficient for a one month medication. Filled aluminium cans formed in this fashion are weight-checked and allowed to rest for an equilibration period before testing.

### Example 2

### (Measurement of particle size distribution and Fine Particle Fraction)

The formulations employed are those formed according to Example 1 above.

The aerodynamic particle size distribution is determined using an Andersen Scale Impactor (ACI) fitted with the universal induction port (as set forth in the USP) at 28.3L/minute.

20 shots (equivalent to 10 doses) of a formulation formed according to Example 1, are discharged into the ACI. Fractions of the dose are deposited at different stages of the ACI, in accordance with the particle size of the fraction. Each fraction is washed from the stage and analysed using HPLC.

HPLC analysis showed that the fine particle fraction of the dose delivered to the ACI apparatus is greater than 50% both for the formoterol fumarate di-hydrate and the beclamethasone dipropionate.

### Example 3

Formulations of Example 1 are tested for Delivered dose Uniformity according to the following method.

Canisters containing formulations are stored at 40°C and 75% rh for 6 months.

After the appropriate storage period, MDI devices containing formulations of Example 1 are connected with the Funnel Apparatus described herein above.

3 doses (6 shots) are discharged into the apparatus at the beginning of the life of the container; 4 doses (8 shots) are discharged in the middle life of the container; and 3 doses (6 shots) are discharged at the end of the container life. The intermediate doses/shots are discharged to waste. The delivered dose is collected by washing the glass scinter, and the dose is analysed by HPLC.

Analysis shows that after the storage period, variance of the delivered dose does not exceed +/- 25% of the mean delivered dose, +/- 20 % of the mean delivered dose.

### Example 4

Formulations of Example 1 are tested for Delivered dose Uniformity according to the following method.

Canisters containing formulations are stored at 40°C and 75% rh for 1, 3 and 6 months.

After the appropriate storage period, MDI devices containing formulations of Example 1 are connected with the Funnel Apparatus described herein above.

For each container, 1 dose (two shots) is discharged into the Funnel Apparatus. This is repeated for 10 containers. After washing the Funnel Apparatus and analysing using HPLC, results show that no delivered dose varies by more than +/- 25% of the mean delivered dose, and more particularly +/-20% of the mean delivered dose.

## Claims

1. A pharmaceutical aerosol formulation for use in a metered dose inhaler (MDI) comprising formoterol fumarate di-hydrate in suspension, a steroid in solution, a propellant and ethanol, wherein the formoterol fumarate di-hydrate has a water content of about 4.8 to 4.28% by weight.

2. A pharmaceutical aerosol suspension formulation according to claim 1 comprising formoterol fumarate di-hydrate in suspension, a steroid in solution, a propellant and ethanol, wherein the formoterol fumarate di-hydrate has a water content of about 4.8 to 4.28% by weight, and wherein the formulation is capable of being dispensed from an MDI to provide a Delivered dose of formoterol fumarate di-hydrate that has a variance of no more than +/- 25%, of the mean Delivered dose when the formulation is stored at 40°C and 75% relative humidity for up to 6 months.

3. A pharmaceutical aerosol suspension formulation according to claim 1 or claim 2 comprising formoterol fumarate di-hydrate in suspension, a steroid in solution, a propellant and ethanol, wherein the formoterol fumarate di-hydrate has a water content of about 4.8 to 4.28% by weight, wherein the formulation is capable of being dispensed from an MDI to provide a Delivered dose of formoterol fumarate di-hydrate with a fine particle fraction of 30 to 70%.

4. A pharmaceutical aerosol suspension formulation according to any of the preceding claims comprising formoterol fumarate di-hydrate in suspension, a steroid in solution, a propellant and ethanol, wherein the formoterol fumarate di-hydrate has a water content of about 4.8 to 4.28% by weight and is provided as particles suspended in the propellant and solvent, and wherein the formulation is capable of being dispensed from an MDI to provide a Delivered dose of the steroid that has a variance of no more than +/- 25%, of the mean Delivered dose when the formulation is stored at 40°C and 75% relative humidity for up to 6 months.

5. A pharmaceutical aerosol suspension formulation according to any of the preceding claims comprising formoterol fumarate di-hydrate in suspension, a steroid in solution, a propellant and ethanol, wherein the formoterol fumarate di-hydrate has a water content of about 4.8 to 4.28% by weight, and wherein the formulation is capable of being dispensed from an MDI to provide a Delivered dose of steroid containing a fine particle fraction of 30% to 70%.

6. A formulation according to any of the preceding claims wherein the steroid is selected from the group consisting of budesonide, ciclesonide, mometasone, fluticasone, beclomethasone, flunisolide, loteprednol, triamcinolone, amiloride, rofleponide and a pharmaceutically acceptable salt or derivative of these active compounds, selected from mometasone furoate, fluticasone dipropionate, beclomethasone dipropionate, triamcinolone acetonide and flunisolide acetate.

7. A formulation according to claim 6 wherein the steroid is ciclesonide.

8. A formulation according to claim 7 wherein the ciclesonide is present in an amount of 0.05 to 2 % by weight of the formulation.

9. A formulation according to any of the preceding claims wherein the formoterol fumarate di-hydrate is present in an amount of 0.001 to 0.1% by weight of the formulation.

10. A formulation according to any of the preceding claims containing a cromone selected from the group consisting of a pharmaceutically acceptable salt of cromoglycinic acid, nedocromil, and mixtures thereof.

11. A formulation according to claim 10 wherein the cromone is present in the formulation in an amount of 0.001 to 1%.

12. A formulation according to any of the preceding claims wherein the propellant is selected from the group consisting of fluorochlorocarbons such as trichloromonofluoromethane (F11), dichlorodifluoromethane (F12), monochlorotrifluoromethane (F13), dichloro-monofluoromethane (F21), monochlorodifluoromethane (F22), monochloromonofluoromethane (F31), 1,1,2-trichloro-1,2,2-trifluoroethane (F113), 1,2-dichloro-1,1,2,2-tetrafluoroethane (F114), 1-chloro-1,1,2,2,2-pentafluoroethane (F 115), 2,2-dichloro-1,1,1-trifluoroethane (F123), 1,2-dichloro-1,1,2-trifluoroethane (F123a), 2-chloro-1,1,1,2-tetrafluoroethane (F124), 2-chloro-1,1,2,2-tetrafluoroethane (F124a), 1,2-dichloro-1,1-difluoroethane (F132b), 1-chloro-1,2,2-trifluoroethane (F133), 2-chloro-1,1,1-trifluoroethane (F133a), 1,1-dichloro-1-fluoroethane (F141b) and 1-chloro-1,1-difluoroethane (F142b), alkanes such as propane, butane and isobutane, fluorinated alkanes such as octafluoropropane (F218) and in particular hydrofluoroalkanes such as difluoromethane (HFA 32), pentafluoroethane (HFA 125), 1,1,2,2-tetrafluoroethane (HFA 134), 1,1,1,2-tetrafluoroethane (HFA 134a), 1,1,2-trifluoroethane (HFA 143), 1,1,1-trifluoroethane (HFA 143a), difluoroethane (HFA 152a), and 1,1,1,2,3,3,3-heptafluoropropane (HFA 227).

13. A formulation according to claim 12 wherein the propellant is a hydrofluoroalkane of the general formula:
CxHyFz (I)
in which x is the number 1, 2 or 3, y and z are each an integer >==1 and y+z=2x+2.

14. A formulation according to claim 12 or claim 13 wherein the propellant is HFA 134a or HFA 227 or a mixture thereof.

15. A formulation according to any of the preceding claims wherein the propellant is employed in an amount of greater than 90% by weight.

16. A formulation according to any of the preceding claims wherein the ethanol is present in an amount of 1% to 8% by weight.

17. A formulation according to any of the preceding claims comprising a surfactant selected from the group consisting of oleic acid, lecithin, sorbitan trioleate, cetylpyridinium chloride, benzalkonium chloride, polyoxyethylene (20) sorbitan monolaurate, polyoxyethylene (20) sorbitan monostearate, polyoxyethylene (20) sorbitan monooleate, polyoxypropylene/polyoxyethylene block copolymers, polyoxypropylene/polyoxyethylene/ethylenediamine block copolymers, and ethoxylated castor oil.

18. A formulation according to claim 17 wherein the surfactant is present in an amount of 0.0001 to 1% by weight.

19. A pharmaceutical aerosol formulation according to claim 1, wherein the moisture content of the formulation is in the range of from 50 ppm to 800 ppm.

20. A vial containing a formulation as defined in any of the preceding claims.

21. A vial according to claim 20 in the form of an aluminium, uncoated container.

22. A vial according to claim 20 or claim 21 adapted to be placed in a metered dose inhaler, and capable of delivering a dosage of formoterol fumarate di-hydrate of about 3 to 15 micro-grams.

23. A vial according to any one of claims 20 to 22 adapted to be placed in a metered dose inhaler, and capable of delivering a dosage of a steroid of about 10 to 1000 micro-grams per puff.

24. A vial according to claim 23 adapted to be placed in a metered dose inhaler, and capable of delivering a dosage of ciclesonide of about 50 to 500 micro-grams per puff.

25. A package comprising a vial as defined in claim 20 or claim 21 containing a formulation as defined in any of the preceding claims, and a label containing a dosage claim, wherein the mean Delivered dose of the active substances is no more than +/-15% of the dosage contained stated in the label.

26. A metered dose inhaler containing a vial as defined in any of claims 20 to 24.

27. A method of producing a pharmaceutical aerosol formulation or a vial as defined in any of claims 1 to 24 comprising the step of drying the formoterol fumarate di-hydrate to a water content of 4.8 to 4.28%.

## Patentansprüche

1. Pharmazeutische Aerosolformulierung zur Verwendung in einem Dosierinhalator (MDI), umfassend Formoterolfumaratdihydrat in Suspension, ein Steroid in Lösung, ein Treibgas und Ethanol, wobei das Formoterolfumaratdihydrat einen Wassergehalt von etwa 4.8 bis 4.28 Gew.-% hat.

2. Pharmazeutische Aerosolsuspensionsformulierung nach Anspruch 1, umfassend Formoterolfumaratdihydrat in Suspension, ein Steroid in Lösung, ein Treibgas und Ethanol, wobei das Formoterolfumaratdihydrat einen Wassergehalt von etwa 4.8 bis 4.28 Gew.-% hat, und wobei die Formulierung so aus einem MDI dosiert werden kann, dass eine verabreichte Dosis (Delivered Dose) Formoterolfumaratdihydrat eine Abweichung von nicht mehr als +/- 25% von der mittleren abgegebenen Dosis hat, wenn die Formulierung bei 40°C und 75% relativer Luftfeuchtigkeit bis zu 6 Monate aufbewahrt wird.

3. Pharmazeutische Aerosolsuspensionsformulierung nach Anspruch 1 oder Anspruch 2, umfassend Formoterolfumaratdihydrat in Suspension, ein Steroid in Lösung, ein Treibgas und Ethanol, wobei das Formoterolfumaratdihydrat einen Wassergehalt von etwa 4.8 bis 4.28 Gew.-% hat, wobei die Formulierung so aus einem MDI dosiert werden kann, dass eine verabreichte Dosis Formoterolfumaratdihydrat einen Feinpartikelanteil von 30 bis 70% hat.

4. Pharmazeutische Aerosolsuspensionsformulierung nach einem der vorhergehenden Ansprüche, umfassend Formoterolfumaratdihydrat in Suspension, ein Steroid in Lösung, ein Treibgas und Ethanol, wobei das Formoterolfumaratdihydrat einen Wassergehalt von etwa 4.8 bis 4.28 Gew.-% hat und in Form von in dem Treibgas und Lösungsmittel suspendierten Partikeln vorliegt, und wobei die Formulierung so aus einem MDI dosiert werden kann, dass eine verabreichte Dosis des Steroids eine Abweichung von nicht mehr als +/- 25% von der mittleren abgegebenen Dosis hat, wenn die Formulierung bei 40°C und 75% relativer Luftfeuchtigkeit bis zu 6 Monate aufbewahrt wird.

5. Pharmazeutische Aerosolsuspensionsformulierung nach einem der vorhergehenden Ansprüche, umfassend Formoterolfumaratdihydrat in Suspension, ein Steroid in Lösung, ein Treibgas und Ethanol, wobei das Formoterolfumaratdihydrat einen Wassergehalt von etwa 4.8 bis 4.28 Gew.-% hat, und wobei die Formulierung so aus einem MDI dosiert werden kann, dass eine verabreichte Dosis Steroid einen Feinpartikelanteil von 30 bis 70% hat.

6. Formulierung nach einem der vorhergehenden Ansprüche, wobei das Steroid ausgewählt ist aus der Gruppe bestehend aus Budesonid, Ciclesonid, Mometason, Fluticason, Beclomethason, Flunisolid, Loteprednol, Triamcinolon, Amilorid, Rofleponid und einem pharmazeutisch verträglichen Salz oder Derivat dieser Wirkstoffe, ausgewählt aus Mometasonfuroat, Fluticasondipropionat, Beclomethasondipropionat, Triamcinolonacetonid und Flunisolidacetat.

7. Formulierung nach Anspruch 6, wobei das Steroid Ciclesonid ist.

8. Formulierung nach Anspruch 7, wobei das Ciclesonid in einer Menge von 0.05 bis 2 Gew.-% der Formulierung vorliegt.

9. Formulierung nach einem der vorhergehenden Ansprüche, wobei das Formoterolfumaratdihydrat in einer Menge von 0.001 bis 0.1 Gew.-% der Formulierung vorliegt.

10. Formulierung nach einem der vorhergehenden Ansprüche enthaltend ein Chromon, ausgewählt aus der Gruppe bestehend aus einem pharmazeutisch verträglichen Salz von Cromoglicinsäure, Nedocromil und Mischungen davon.

11. Formulierung nach Anspruch 10, wobei das Chromon in der Formulierung in einer Menge von 0.001 bis 1% vorliegt.

12. Formulierung nach einem der vorhergehenden Ansprüche, wobei das Treibgas ausgewählt ist aus der Gruppe bestehend aus Fluorchlorkohlenwasserstoffen wie Trichlormonofluormethan (F11), Dichlordifluormethan (F12), Monochlortrifluormethan (F13), Dichlormonofluormethan (F21), Monochlordifluormethan (F22), Monochlormonofluormethan (F31), 1,1,2-Trichlor-1,2,2-trifluorethan (F113), 1,2-Dichlor-1,1,2,2-tetrafluorethan (F114), 1-Chlor-1,1,2,2,2-Pentafluorethan (F115); 2,2-Dichlor-1,1,1-trifluorethan (F123), 1,2-Dichlor-1,1,2-trifluorethan (F123a), 2-Chlor-1,1,1,2-tetrafluorethan (F124), 2-Chlor-1,1,2,2-tetrafluorethan (F124a), 1,2-Dichlor-1,1-difluorethan (F132b), 1-Chlor-1,2,2-trifluorethan (F133), 2-Chlor-1,1,1-trifluorethan (F133a), 1,1-Dichlor-1-fluorethan (F141b) und 1-Chlor-1,1-difluorethan (F142b), Alkanen wie Propan, Butan und Isobutan, fluorierten Alkanen wie Octafluorpropan (F218) und insbesondere Hydrofluoralkanen wie Difluormethan (HFA 32), Pentafluorethan (HFA 125), 1,1,2,2-Tetrafluorethan (HFA 134), 1,1,1,2-Tetrafluorethan (HFA 134a), 1,1,2-Trifluorethan (HFA 143), 1,1,1-Trifluorethan (HFA 143a), Difluorethan (HFA 152a) und 1,1,1,2,3,3,3-Heptafluorpropan (HFA 227).

13. Formulierung nach Anspruch 12, wobei das Treibgas ein Hydrofluoralkan mit der allgemeinen Formel
CxHyFz (I)
ist, wobei x die Zahl 1, 2 oder 3 ist, y und z jeweils eine ganze Zahl >==1 und y+z=2x+2.

14. Formulierung nach Anspruch 12 oder Anspruch 13, wobei das Treibgas HFA 134a, HFA 227 oder eine Mischung davon ist.

15. Formulierung nach einem der vorherigen Ansprüche, wobei das Treibgas in einer Menge von mehr als 90 Gew.-% eingesetzt wird.

16. Formulierung nach einem der vorhergehenden Ansprüche, wobei das Ethanol in einer Menge von 1 bis 8 Gew.-% vorliegt.

17. Formulierung nach einem der vorhergehenden Ansprüche, umfassend ein Tensid, welches ausgewählt ist aus der Gruppe bestehend aus Ölsäure, Lecithin, Sorbitantrioleat, Cetylpyridiniumchlorid, Benzalkoniumchlorid, Polyoxyethylen(20)-sorbitanmonolaurat, Polyoxyethylen(20)-sorbitanmonostearat, Polyoxyethylen(20)-sorbitanmonooleat, Polyoxypropylen/Polyoxyethylen-Blockcopolymeren, Polyoxypropylen/Polyoxyethylen/Ethylendiamin-Blockcopolymeren und ethoxyliertem Castoröl.

18. Formulierung nach Anspruch 17, wobei das Tensid in einer Menge von 0.0001 bis 1 Gew.-% vorliegt.

19. Pharmazeutische Aerosolformulierung nach Anspruch 1, wobei der Feuchtigkeitsgehalt der Formulierung im Bereich von 50 ppm bis 800 ppm liegt.

20. Ampulle, welche eine Formulierung gemäß einem der vorhergehenden Ansprüche enthält.

21. Ampulle nach Anspruch 20 in Form eines unbeschichteten Aluminiumbehälters,

22. Ampulle nach Anspruch 20 oder Anspruch 21, die so ausgelegt ist, dass sie in einen Dosierinhalator eingesetzt werden kann und eine Dosierung Formoterolfumaratdihydrat von etwa 3 bis 15 Mikrogramm abgeben kann.

23. Ampulle nach einem der Ansprüche 20 bis 22, die so ausgelegt ist, dass sie in einen Dosierinhalator eingesetzt werden kann und eine Dosierung eines Steroids von etwa 10 bis 1000 Mikrogramm pro Sprühstoß abgeben kann.

24. Ampulle nach Anspruch 23, die so ausgelegt ist, dass sie in einen Dosierinhalator eingesetzt werden kann und eine Dosierung Ciclesonid von etwa 50 bis 500 Mikrogramm pro Sprühstoß abgeben kann.

25. Packung, welche eine Ampulle nach Anspruch 20 oder Anspruch 21 umfasst, welche eine Formulierung gemäß einem der vorhergehenden Ansprüche und einen Beipackzettel mit einer Dosierungsangabe enthält, wobei die mittlere verabreichte Dosis der Wirkstoffe nicht mehr als +/- 15% von der enthaltenen Dosis abweicht, die auf dem Beipackzettel angegeben ist.

26. Dosierinhalator, welcher eine Ampulle gemäß einem der Ansprüche 20 bis 24 enthält.

27. Verfahren zur Herstellung einer pharmazeutischen Aerosolformulierung oder einer Ampulle gemäß einem der Ansprüche 1 bis 24, umfassend den Schritt des Trocknens des Formoterolfumaratdihydrats bis zu einem Wassergehalt von 4.8 bis 4.28%.

## Revendications

1. Formulation pharmaceutique en aérosol pour utilisation dans un inhalateur doseur (MDI), comprenant du fumarate de formotérol dihydraté en suspension, un stéroïde en solution, un propulseur et de l'éthanol, dans laquelle le fumarate de formotérol dihydraté présente une teneur en eau d'environ 4,8 à 4,28 % en poids.

2. Formulation pharmaceutique en aérosol en suspension selon la revendication 1, comprenant du fumarate de formotérol dihydraté en suspension, un stéroïde en solution, un propulseur et de l'éthanol, dans laquelle le fumarate de formotérol dihydraté présente une teneur en eau d'environ 4,8 à 4,28 % en poids, laquelle formulation peut être délivrée à partir d'un MDI pour délivrer une dose de fumarate de formotérol dihydraté présentant une variance ne dépassant pas ± 25 % de la dose délivrée moyenne quand la formulation est stockée à 40°C et sous une humidité relative de 75 % pendant jusqu'à 6 mois.

3. Formulation pharmaceutique en aérosol en suspension selon la revendication 1 ou 2, comprenant du fumarate de formotérol dihydraté en suspension, un stéroïde en solution, un propulseur et de l'éthanol, dans laquelle le fumarate de formotérol dihydraté présente une teneur en eau d'environ 4,8 à 4,28 % en poids, laquelle formulation peut être délivrée à partir d'un MDI pour délivrer une dose de fumarate de formotérol dihydraté présentant une fraction de fines particules de 30 à 70 %.

4. Formulation pharmaceutique en aérosol en suspension selon l'une quelconque des revendications précédentes, comprenant du fumarate de formotérol dihydraté en suspension, un stéroïde en solution, un propulseur et de l'éthanol, dans laquelle le fumarate de formotérol dihydraté présente une teneur en eau d'environ 4,8 à 4,28 % en poids et se présente sous la forme de particules en suspension dans le propulseur et le solvant, laquelle formulation peut être délivrée à partir d'un MDI pour délivrer une dose du stéroïde présentant une variance ne dépassant pas ± 25 % de la dose délivrée moyenne quand la formulation est stockée à 40°C et sous une humidité relative de 75 % pendant jusqu'à 6 mois.

5. Formulation pharmaceutique en aérosol en suspension selon l'une quelconque des revendications précédentes, comprenant du fumarate de formotérol dihydraté en suspension, un stéroïde en solution, un propulseur et de l'éthanol, dans laquelle le fumarate de formotérol dihydraté présente une teneur en eau d'environ 4,8 à 4,28 % en poids, laquelle formulation peut être délivrée à partir d'un MDI pour délivrer une dose de stéroïde contenant une fraction de fines particules de 30 à 70 %.

6. Formulation pharmaceutique en aérosol en suspension selon l'une quelconque des revendications précédentes, dans laquelle le stéroïde est choisi dans l'ensemble constitué par le budésonide, le ciclésonide, la mométasone, la fluticasone, la béclométhasone, le flunisolide, le lotéprednol, la triamcinolone, l'amiloride, le rofléponide et un sel ou dérivé pharmaceutiquement acceptable de ces agents actifs, choisi parmi le furoate de mométasone, le dipropionate de fluticasone, le dipropionate de béclométhasone, l'acétonide de triamcinolone, et l'acétate de flunisolide.

7. Formulation selon la revendication 6, dans laquelle le stéroïde est le ciclésonide.

8. Formulation selon la revendication 7, dans laquelle le ciclésonide est présent en une quantité de 0,05 à 2 % en poids de la formulation.

9. Formulation selon l'une quelconque des revendications précédentes, dans laquelle le fumarate de formotérol dihydraté est présent en une quantité de 0,001 à 0,1 % en poids de la formulation.

10. Formulation selon l'une quelconque des revendications précédentes, contenant une cromone choisie dans l'ensemble constitué par un sel pharmaceutiquement acceptable d'acide cromoglycinique, le nédocromil, et leurs mélanges.

11. Formulation selon la revendication 10, dans laquelle la cromone est présente dans la formulation en une quantité de 0,001 à 1 %.

12. Formulation selon l'une quelconque des revendications précédentes, dans laquelle le propulseur est choisi dans l'ensemble constitué par les fluorochlorocarbures tels que le trichloromonofluoro-méthane (F11), le dichlorodifluorométhane (F12), le monochlorotrifluorométhane (F13), le dichloromonofluoro-méthane (F21), le monochlorodifluorométhane (F22), le monochloromonofluorométhane (F31), le 1,1,2-trichloro-1,2,2-trifluoroêthane (F113), le 1,2-dichloro-1,1,2,2-tétrafluoroéthane (F114), le 1-ohloro-1,1,2,2,2-pentafluoroéthane (F115), le 2,2-dichloro-1,1,1-trifluoroéthane (F123), le 1,2-dchloro-1,1,2-trifluoroéthane (F123a), le 2-chloro-1,1,1,2-tétrafluoroéthane (F124), le 2-chloro-1,1,2,2-tétrafluoroéthane (F124a), le 1,2-dichloro-1,1-difluoroéthane (F132b), le 1-chloro-1,2,2-trifluoroéthane (F133), le 2-chloro-1,1,1-trifluoroéthane (F133a), le 1,1-dichloro-1-fluoroéthane (F141b) et le 1-chloro-1,1-difluoroéthane (F142b), les alcanes tels que le propane, le butane et l'isobutane, les alcanes fluorés tels que l'octafluoropropane (F128) et en particulier les hydrofluoroalcanes tels que le difluorométhane (HFA 32), le pentafluoroéthane (HFA 125), le 1,1,2,2-tétrafluoroéthane (HFA 134), le 1,1,1,2-tétrafluoroéthane (HFA 134a), le 1,1,2-trifluoroéthane (HFA 143), le 1,1,1-trifluoroéthane (HFA 143a), le difluoroéthane (HFA 152a), et le 1,1,1,2,3,3,3-heptafluoropropane (HFA 227).

13. Formulation selon la revendication 12, dans laquelle le propulseur est un hydrofluoroalcane de formule générale :
CxHyFz (I)
dans laquelle x est le nombre 1, 2 ou 3, et chacun de y et z est un entier ≥ 1, et y + z = 2x + 2.

14. Formulation selon la revendication 12 ou la revendication 13, dans laquelle le propulseur est le HFA 134a ou le HFA 227 ou un mélange de ceux-ci.

15. Formulation selon l'une quelconque des revendications précédentes, dans laquelle le propulseur est employé en une quantité supérieure à 90 % en poids.

16. Formulation selon l'une quelconque des revendications précédentes, dans laquelle l'éthanol est présent en une quantité de 1 % à 8 % en poids.

17. Formulation selon l'une quelconque des revendications précédentes, comprenant un tensioactif choisi dans l'ensemble constitué par l'acide oléique, la lécithine, le trioléate de sorbitan, le chlorure de cétylpyridinium, le chlorure de benzalkonium, le monolaurate de sorbitan polyoxyéthyléné (20), le monostéarate de sorbitan polyoxyéthyléné (20), le monooléate de sorbitan polyoxyéthyléné (20), les copolymères séquencés de poly(oxyde de propylène)/ poly(oxyde d'éthylène), les copolymères séquencés de poly(oxyde de propylène)/poly(oxyde d'éthylène)/ éthylènediamine, et l'huile de ricin éthoxylée.

18. Formulation selon la revendication 17, dans laquelle le tensioactif est présent en une quantité de 0,0001 à 1 % en poids.

19. Formulation pharmaceutique en aérosol selon la revendication 1, dans laquelle la teneur en humidité de la formulation est située dans la plage allant de 50 ppm à 800 ppm.

20. Flacon contenant une formulation telle que définie dans l'une quelconque des revendications précédentes.

21. Flacon selon la revendication 20, sous la forme d'un récipient en aluminium non revêtu.

22. Flacon selon la revendication 20 ou la revendication 21, adapté pour être placé dans un inhalateur doseur, et capable de délivrer une dose de fumarate de formotérol dihydraté d'environ 3 à 15 microgrammes.

23. Flacon selon l'une quelconque des revendications 20 à 22, adapté pour être placé dans un inhalateur doseur, et capable de délivrer une dose de stéroïde d'environ 10 à 1000 microgrammes par bouffée.

24. Flacon selon la revendication 23 adapté pour être placé dans un inhalateur doseur, et capable de délivrer une dose de ciclésonide d'environ 50 à 500 microgrammes par bouffée.

25. Conditionnement comprenant un flacon tel que défini dans la revendication 20 ou la revendication 21 contenant une formulation telle que définie dans l'une quelconque des revendications précédentes, et une étiquette contenant une déclaration de quantité, dans lequel la dose délivrée moyenne des substances actives ne dépasse pas ± 15 % de la dose contenue indiquée sur l'étiquette.

26. Inhalateur doseur contenant un flacon tel que défini dans l'une quelconque des revendications 20 à 24.

27. Procédé pour produire une formulation pharmaceutique en aérosol ou un flacon tel que défini dans l'une quelconque des revendications 1 à 24, comprenant l'étape consistant à sécher le fumarate de formotérol dihydraté jusqu'à une teneur en eau de 4,8 à 4,28 %.
